# EUROPÄISCHE PATENTSCHRIFT

(11) **EP 2 678 053 B1**
(45) Veröffentlichungstag und Bekanntmachung des Hinweises auf die Patenterteilung: **17.05.2017**
(21) Anmeldenummer: 12705307.2
(22) Anmeldetag: 23.02.2012
(51) Int. Cl.: A61M 1/14, A61M 1/36

(54) **VORRICHTUNG ZUM UNTERSTÜTZEN EINES BEDIENERS BEI DER BEDIENUNG EINES MEDIZINISCHEN GERÄTS**
DEVICE FOR ASSISTING AN OPERATOR IN OPERATING A MEDICAL APPLIANCE
DISPOSITIF POUR ASSISTER UN OPÉRATEUR LORS DU FONCTIONNEMENT D'UN APPAREIL MÉDICAL

(30) Priorität: 23.02.2011 DE 102011004620; 23.02.2011 US 201161445597 P
(43) Veröffentlichungstag der Anmeldung: 01.01.2014
(73) Patentinhaber: Fresenius Medical Care Deutschland GmbH, 61352 Bad Homburg (DE)
(72) Erfinder: THYS, Martin, 97508 Grettstadt (DE)
(74) Vertreter: Nordmeyer, Philipp Werner
(86) Internationale Anmeldenummer: PCT/EP2012/053057
(87) Internationale Veröffentlichungsnummer: WO 2012/113858

(56) Entgegenhaltungen:
- EP-A1- 1 676 539
- EP-A1- 1 872 814
- EP-A2- 1 195 171
- US-A1- 2007 106 263
- US-A1- 2009 012 449
- US-A1- 2009 294 339

## Beschreibung

### Technisches Gebiet

Die vorliegende Erfindung betrifft eine Vorrichtung und ein Verfahren zum Unterstützen eines Bedieners bei der Bedienung eines medizinischen Geräts, ein medizinisches Gerät, sowie einen Einmalartikel für ein medizinisches Gerät.

### Technischer Hintergrund

Die Bedienung medizinischer Geräte mit all ihren Komponenten ist häufig komplex und kann erst nach Schulungen beziehungsweise aufwändigem Selbststudium von Bedienungsanleitungen sicher durchgeführt werden. Dies ist beispielsweise im Bereich der Blutbehandlungsgeräte, insbesondere bei Dialysevorrichtungen wie beispielsweise Hämodialysevorrichtungen, Peritonealdialysevorrichtungen, Hämofiltrationsvorrichtungen oder Hämodiafiltrationsvorrichtungen, Apheresevorrichtungen oder auch bei anderen medizinischen Geräten beispielsweise zur Verabreichung einer Medikation der Fall.

Bei diesen medizinischen Geräten müssen häufig Einmalartikel ausgetauscht werden, welche bestimmte, mit dem medizinischen Gerät in Verbindung stehende Komponenten umfassen. Beispiele solcher Einmalartikel sind Zuführschläuche für das Patientenblut, Beutel für Dialysat, Filter in einer Dialysevorrichtung oder Spritzen zum Dosieren von Medikamenten, welche mit dem medizinischen Gerät in Verbindung stehende Komponenten in Form von Schlauchanschlüssen, Halterungen für Filter, Schlauchabschnitte zum Einbringen in Quetschpumpen, Spritzentüllen, Spritzenkolben etc. aufweisen. Zum Austausch dieser Einmalartikel in dem medizinischen Gerät und zur Bedienung der einzelnen Komponenten der jeweiligen Einmalartikel muss häufig eine bestimmte Reihenfolge der durchzuführenden Handgriffe eingehalten werden.

Obwohl sich die entsprechenden medizinischen Geräte unterschiedlicher Bauarten im Grundsatz unterscheiden, sind doch die zu bedienenden Komponenten häufig ähnlich. Entsprechend kann sich ein Bediener, der mit der grundlegenden Funktion eines solchen medizinischen Geräts vertraut ist, auch auf einem ähnlichen medizinischen Gerät anderer Bauart relativ schnell durch Studium einer Kurzanleitung zurechtfinden.

Bei den genannten medizinischen Geräten, insbesondere bei medizinischen Geräten zur Blutbehandlung sind zumindest vor und/oder während und/oder nach der Behandlung des jeweiligen Patienten Interaktionen eines Bedieners mit dem medizinische Gerät notwendig, um dieses für die Behandlung des Patienten herzurichten, den Betrieb aufrechtzuerhalten und/oder das medizinische Gerät nach der Behandlung wieder in einen Ausgangszustand zur Durchführung einer Behandlung eines weiteren Patienten zu versetzen.

Beispielsweise müssen bei Blutbehandlungsvorrichtungen Verbindungen des medizinischen Geräts mit dem Blutkreislauf des Patienten hergestellt werden, was typischerweise die Verbindung jeweils eines Schlauches mit dem venösen und dem arteriellen Zugang des Patienten beinhaltet. Weiterhin müssen in die Blutbehandlungsvorrichtung vom Blut durchflossene Einmalartikel bzw. Verbrauchsartikel eingelegt werden und nach der Behandlung wieder entfernt werden, die Blutbehandlungsvorrichtung muss mit entsprechenden Flüssigkeitszufuhren und Gaszufuhren versorgt werden und Hilfsstoffe müssen zu- beziehungsweise abgeführt werden. Bei den mit dem Patienten direkt verbundenen Komponenten handelt es sich typischerweise um diese Komponenten umfassende Einmalartikel beziehungsweise Wegwerfartikel, die für jeden Patienten neu angebracht werden müssen und die nach der Behandlung entfernt und entsorgt werden. Diese Einmalartikel sind beispielsweise die Schläuche, die den Blutkreislauf des Patienten mit dem medizinischen Gerät verbinden, die Zuführungen innerhalb des medizinischen Geräts, die Filtermembran sowie die Beutel zur Aufnahme des Dialysats. Bei der Bedienung des medizinischen Geräts müssen entsprechend bei jedem neuen Patienten Routineschritte durchgeführt werden, die nach einer Schulung des jeweiligen Bedienpersonals von diesem einfach durchgeführt werden können.

Allerdings muss für jede Bauarttype eine eigene Schulung durchgeführt werden. Hierdurch kann es leicht zu Verwechselungen oder Verwirrungen beim Bediener kommen, wenn ein Bediener medizinische Geräte unterschiedlicher Bauart nacheinander beziehungsweise gleichzeitig zu betreuen hat.

Für die teilweise sehr umfangreichen Bedienprozeduren ist entsprechend häufig speziell geschultes Fachpersonal notwendig, da nur dieses bei zeitkritischen Bedienaktionen an dem medizinischen Gerät die erforderlichen Kenntnisse, Fähigkeiten und die Praxiserfahrung besitzt, um gegebenenfalls auch ohne ein erneutes Studium der Bedienungsanleitung die korrekte Bedienaktion vornehmen zu können. In diesem Zusammenhang ist es bekannt, an einem Gerätebildschirm des medizinischen Geräts entsprechende text- und bildbasierte Bedienungshinweise auszugeben. Bei solchen text- oder bildbasierten Bedienungshinweisen, die von dem medizinischen Gerät auf einem Monitor ausgegeben werden, ist es jedoch notwendig, die einzelnen Komponenten, an denen die Bedienschritte durchgeführt werden sollen, entweder abzubilden oder sie mit einer Bedeutung zu versehen. Der Bediener muss dann die eigentlich zu bedienende Komponente mit dem Bild und/oder der in Textform angegebenen Anleitung korrelieren. Dabei kann es zu schwerwiegenden Fehlern kommen, da Texte und/oder Bilder auf dem Gerätemonitor fehlinterpretiert werden können und entsprechend an einer anderen Komponente, als der eigentlich gemeinten, Bedienaktionen durchgeführt werden.

Weiterhin ist bei für die Heimanwendung vorgesehenen medizinischen Geräten nicht davon auszugehen, dass ein Patient selbst das Fachwissen aufbaut, welches für die Bedienung eines medizinischen Geräts notwendig sein kann.

Entsprechend sollte sich ein medizinisches Gerät nach möglichst kurzer Einarbeitung durch einen Bediener sicher und intuitiv bedienen lassen. Es wäre wünschenswert, wenn die einzelnen durchzuführenden Bedienprozeduren von jedem Bediener, unabhängig von dem jeweiligen Erfahrungs- und Wissensstand, stets sicher und vollständig durchgeführt werden könnten. Da die medizinischen Geräte auf den Eingriff von Bedienern, beispielsweise zum Austausch der Einwegkomponenten, angewiesen sind, kann auch auf eine Interaktion zwischen einem Bediener und dem medizinischen Gerät keinesfalls verzichtet werden.

Die EP 1 872 814 A1 betrifft ein medizintechnisches System zur Durchführung einer Fluidbehandlung eines Patienten. Dabei ist ein elektronisches Lesesystem mit mindestens einem Lesegerät vorgesehen, vor das ein Bediener einen Verbinder eines Fluidbeutels bringen kann, um dann mittels einer LED einen Anschluss angezeigt zu bekommen, an welchen der Verbinder für das jeweilige Behandlungsprogramm angeschlossen werden muss.

Die US 2007/106263 A1 betrifft eine intravenöse Identifikationsbeleuchtung und eine Lichtröhre.

Die US 2009/012449 A1 betrifft ein Fludizuführsystem mit automatischer Verbindungsherstellung.

Die EP 1 195 171 A2 betrifft eine Vorrichtung zur Peritoneal-Dialyse.

Die EP 1 676 539 A1 betrifft ein System und ein Verfahren zum Sicherstellen einer richtigen Instrumentenhandhabung in einem Operationsraum.

### Beschreibung der Offenbarung

Aufgabe der vorliegenden Erfindung ist es daher, eine Vorrichtung zur Unterstützung eines Bedieners beim Bedienen eines medizinischen Geräts anzugeben, bei welcher die Sicherheit der Bedienung des medizinischen Geräts erhöht wird.

Diese Aufgabe wird gelöst durch eine Blutbehandlungsvorrichtung mit den Merkmalen des Anspruchs 1 und durch eine Blutbehandlungsvorrichtung mit den Merkmalen des Anspruchs 4. Bevorzugte Ausgestaltungen sind in den Unteransprüchen angegeben.

Entsprechend umfasst die Vorrichtung eine Signalisiervorrichtung zum Signalisieren einer erforderlichen Bedienung einer zu bedienenden Komponente in Form des Systemschlauchs des Anspruchs 1 oder des Klemmhebels des Anspruchs 4 der Blutbehandlungsvorrichtung.

Durch die Verwendung der Signalisiervorrichtung zum Signalisieren einer erforderlichen Bedienung einer zu bedienenden Komponente wird der Bediener auf eine zu bedienenden Komponente aufmerksam gemacht und zu dieser hingeführt, so dass der Bediener diese zu bedienende Komponente unmittelbar in den nächsten Bedienschritt mit einbezieht. Dabei wird erreicht, dass die Aufmerksamkeit des Bedieners auf die zu bedienenden Komponente gelenkt wird und die Gefahr einer Fehlbedienung reduziert wird.

Es ergibt sich daher bei der Bedienung des medizinischen Geräts quasi eine intuitive Bedienung, die entsprechend die jeweilige Bedienung durch die subjektive Wahrnehmung der von der Signalisiervorrichtung ausgegebenen Signalisierung veranlasst, ohne dass der Bediener kognitive Anstrengungen unternehmen muss. Der Bediener wird entsprechend durch die suggestiv / intuitiv sofort erfassbare Signalisierung zu einem Handlungsschritt beziehungsweise zu einer Aktion veranlasst und kann die korrekte Bedienung des medizinischen Geräts durchführen. Dies ist sowohl unter Sicherheitsaspekten wesentlich, also auch unter Effizienzgesichtspunkten, da eigene Überlegungen durch den Bediener, insbesondere bei Standardroutinen, weitgehend ausgeschaltet werden können und der Bediener die korrekte Bedienung spontan und sofort durchführt. Im Gegensatz hierzu benötigen logische Überlegungen und das Ziehen von Schlussfolgerungen Zeit, und solche Überlegungen beziehungsweise das Ziehen von Schlussfolgerungen sind insbesondere unter dem, im medizinischen Bereich häufig vorhandenen, Zeitdruck oder Stress fehlerträchtig.

Durch die Signalisierung der zu bedienenden Komponenten durch die Signalisiervorrichtung wird weiterhin sichergestellt, dass auch bei Nicht-Vorliegen einer Bedienungsanleitung, in welcher die notwendigen Bedienschritte dokumentiert sind, die erforderlichen Bedienschritte dennoch durchgeführt werden können. Eine ständige Verfügbarkeit von Bedienungsanleitungen ist nicht immer gegeben, da diese manchmal örtlich getrennt von dem eigentlichen medizinischen Gerät aufbewahrt werden. Es sind jedoch bei medizinischen Geräten, insbesondere bei den oben genannten Blutbehandlungsvorrichtungen bestimmte Bedienprozeduren sowohl in ihrer Reihenfolge als auch in ihrer Vollständigkeit strikt einzuhalten, da diese sicherheitsrelevant sind und eine fehlerhafte Bedienung eine Schädigung des zu behandelnden Patienten, der Umgebung und/oder der Vorrichtung nach sich ziehen könnte. Weiterhin ist das Vorhalten von gut geschultem Fachpersonal kostenintensiv und daher unter ökonomischen Gesichtspunkten ungünstig.

Durch die Signalisiervorrichtung und deren gezielter Ansteuerung bei der Notwendigkeit einer durchzuführenden Bedienung wird eine Vereinfachung bei der Bedienung des medizinischen Geräts erreicht, da die erreichte intuitivere Bedienung beim Bediener gerade in zeitkritischen oder stressbehafteten Situationen deutlich schneller und intuitiver durchgeführt werden kann und entsprechend die Gefahr einer Fehlbedienung reduziert werden kann. Entsprechend werden die kognitiven Anforderungen an den Bediener, was die Merkfähigkeit, das Kombinationsvermögen und das Konzentrationsvermögen betrifft, reduziert. Insbesondere können die Schritte zur mechanischen Vorbereitung eines medizinischen Geräts, wie beispielsweise das Einlegen der jeweiligen Einmalkomponenten in das medizinische Gerät, wie auch die Nachbereitung des medizinischen Geräts, nämlich das Entnehmen und Entsorgen der Einmalkomponenten, auch von weitgehend ungeschultem Personal durchgeführt werden.

Weiterhin ist die Signalisierung durch die Signalisiervorrichtung an der zu bedienenden Komponente deshalb von Vorteil, da der Bediener sich vollständig auf die teilweise anspruchsvollen und komplizierten mechanischen Manipulationen der zu bedienenden Komponente des medizinischen Geräts konzentrieren kann, da sein Blick nicht von der Komponente gelöst werden muss, sondern durch die Signalisierung an der Komponente sowohl das Signal als auch die Komponente selbst gleichzeitig im Blickfeld behalten werden können.

In einem Fall, in welchem der Bediener die richtige Bedienung beziehungsweise Manipulation einer Komponente durch Überprüfung eines Zustands an einem anderen Ort als der Komponente selbst überprüfen können muss, kann die Komponente selbst weiterhin haptische Informationen aussenden, beispielsweise Vibrieren, um dem Bediener einen bestimmten Bedienzustand der Komponenten zu übermitteln, während er den Blick woandershin gerichtet hat. Beispielsweise kann der Bediener beim Zusammenstecken zweier Komponenten in einer bestimmten Orientierung die Dichtheit der Komponenten auf der Systemanzeige überprüfen. Hier kann eine haptische Rückmeldung über eine Vibration geliefert werden, wenn die Komponenten in der richtigen Orientierung zusammengefügt sind, während der Bediener auf der Systemanzeige die Dichtheit der zusammengefügten Komponenten überprüft.

Unter zu bedienender Komponenten wird vorliegend eine Komponente des medizinischen Geräts verstanden, welche einem Bedienschritt durch einen Bediener unterzogen werden muss. Dies können zum einen Eingabevorrichtungen, wie beispielsweise Knöpfe, Schalter, Touchscreens etc. sein, zum anderen aber vor allem mechanische Komponente, wie beispielsweise die Bedienung von Schlauchklemmen, Pumpen etc.. Weiterhin sind darunter Komponenten von Einmalartikeln wie beispielsweise Zuführungsschläuche, Filter, Medienzuführungen, Dialysatbeutel, Spritzen etc., zu verstehen, welche als zu bedienende Komponenten entsprechende Anschlussstücke, Tüllen, Schlauchabschnitte, Klemmen etc. umfassen. Unter den zu bedienenden Komponenten werden also sämtliche zu bedienende Komponenten, welche für den Betrieb des medizinischen Geräts notwendig sind, verstanden.

Die Signalisiervorrichtung kann einen optischen, haptischen und/oder akustischen Signalgeber zur Erzeugung eines optischen, haptischen und/oder akustischen Signals umfassen. Auf diese Weise kann an der jeweils zu bedienenden Komponente selektiv ein optisches, haptisches und/oder akustisches Signal ausgegeben werden. Weiterhin kann durch die Anordnung entsprechender optischer, haptischer oder akustischer Signalgeber an der Komponente selbst mittels der Signalisiervorrichtung eine Information ausgegeben werden, beispielsweise mittels eines Symbols oder unterschiedlicher Symbole, mittels unterschiedlicher akustischer oder haptischer Signale oder mittels der Anzeige von Text. Diese Signale können mit den an einer Geräteanzeige des medizinischen Geräts ausgegebenen Informationen korreliert sein. Insbesondere kann der Bediener zusätzliche Informationen, beispielsweise Texte, an der Geräteanzeige lesen und diese mit einem an der jeweiligen Komponente signalisierten Symbol in Beziehung setzen.

Die Signalisiervorrichtung kann ein Projektor sein, welcher die zu bedienende Komponente derart beleuchtet, dass die zu bedienende Komponente ein Signal in Form des reflektierten Lichtes aussendet. Hierüber kann effizient eine Beleuchtung und damit das Aussenden eines Signals erreicht werden. Die Signalisiervorrichtung kann auch eine in der zu bedienenden Komponente vorgesehene Lichtquelle sein, bevorzugt eine LED. Weiterhin kann die Signalisiervorrichtung ein elektrodynamischer oder elektroakustischer Wandler sein, welcher mit der zu bedienenden Komponente wechselwirkt und/oder an dieser vorgesehen ist. Zumindest in dem Falle des Projektors als auch im Falle des elektrodynamischen Wandlers kann die Signalisiervorrichtung mit der zu bedienenden Komponente wechselwirkten, um das Signal von der Komponente auszusenden.

Um die intuitive Information des Bedieners bezüglich der zu bedienenden Komponenten zu erreichen, gehen die Signale der Signalisiervorrichtung von der zu bedienenden Komponente aus. Dies kann entweder dadurch erreicht werden, dass die Komponente selbst ein optisches, haptisches oder akustisches Signal aussendet, indem sie beispielsweise selbst mit einer Lichtquelle oder einer optischen Vorrichtung derart ausgebildet ist, dass eingekoppeltes Licht an einer bestimmten Stelle der Komponente aus der Komponente wieder austritt, dass ein Vibrationsgeber in der Komponente vorgesehen ist oder ein akustischer Signalgeber, oder aber dadurch, dass die zu bedienende Komponente von außen mittels einer Beleuchtungsvorrichtung gezielt beleuchtet wird und die Komponente durch Reflektion des Lichtes ein Signal aussendet.

Besonders im Bereich der Einmalartikel kann die Komponente einen Lichtleiter umfassen und/oder als Lichtleiter ausgebildet sein, wobei über die Signalisiervorrichtung Licht in die Komponente einkoppelbar ist und aus dem Einmalartikel entsprechend ein optisches Signal ausgekoppelt werden kann, um an den Benutzer ein Signal zu senden. Auf diese Weise kann auf eine aufwändige Konstruktion der Einmalartikel verzichtet werden.

Um dem Benutzer bestimmte Informationen zukommen zu lassen, kann die Signalisiervorrichtung zur Aktivierung mindestens eines an der zu bedienenden Komponente vorgesehenen optischen Symbols ausgebildet sein. Bevorzugt ist die Signalisiervorrichtung zur wahlweisen Aktivierung von mindestens zwei an der zu bedienenden Komponente angeordneten optischen Symbolen ausgebildet. Auf diese Weise können auch sich ändernde Zustände, beispielsweise der Übergang von einer unvollständigen Verriegelung zweier Komponenten hin zu einer vollständigen Verriegelung zweier Komponenten, dem Benutzer kommuniziert werden.

Mittels einer Steuervorrichtung zur Ansteuerung der Signalisiervorrichtung in Abhängigkeit von der durchzuführenden Bedienung kann dem Benutzer eine Bedienreihenfolge unterschiedlicher Komponenten vorgegeben werden. Dies ist bevorzugt unabhängig von einer Benutzeranforderung - der Benutzer muss die Steuervorrichtung also zur Ausgabe der Signalisierung nicht auffordern, sondern es ist die Steuervorrichtung, die den Benutzer zur Durchführung einer Aktion auffordert. Zu diesem Zwecke ist die Steuervorrichtung bevorzugt mit einem Systemmonitor des medizinischen Geräts verbunden und kann entsprechend der Systemzustände des medizinischen Geräts reagieren und den Benutzer zu entsprechenden Aktionen auffordern.

wahlweise aktivierbare optische Symbole. Die Komponente kann als Einmalartikel ausgebildet sein und kann bevorzugt als Verbrauchsartikel des medizinischen Geräts ausgebildet sein.

Bevorzugt ist die Signalisiervorrichtung ein Teil des medizinischen Geräts und insbesondere entweder in den Hauptteil des medizinischen Geräts integriert, in einen Einmalartikel integriert, oder als von den üblichen Einmalartikeln separater und lösbarer Artikel vorliegend vorgesehen.

### Kurze Beschreibung der Figuren

Im Folgenden werden bevorzugte Ausführungsformen der vorliegenden Erfindung anhand der nachfolgenden Figuren beschrieben:
- Figur 1: zeigt eine Komponente in Form eines Blutschlauches, welcher in dem medizinischen Gerät gehalten ist;
- Figur 2: zeigt eine weitere Komponente in Form einer Schlauchklemmvorrichtung, welche manuell betätigt werden muss, mit eingelegtem Systemschlauch;
- Figur 3: zeigt eine alternative Ausführungsform der in Figur 2 gezeigten Schlauchklemmeinrichtung;
- Figur 4: zeigt eine Schlauchklemmeinrichtung in einer weiteren Ausführungsform;
- Figur 5: zeigt eine weitere Schlauchklemmvorrichtung mit einer Signalisiervorrichtung;
- Figur 6: zeigt eine Komponente in Form eines Systemschlauchs schematisch im Querschnitt und in Draufsicht;
- Figur 7: zeigt den Systemschlauch aus Figur 6 in einer Ausschnittsdarstellung;
- Figur 8: zeigt eine Schlauchklemmeinrichtung in einem weiteren Ausführungsbeispiel und
- Figur 9: zeigt eine Schnittstelle zwischen einer Behandlungsvorrichtung und einem Einmalartikel.

### Ausführliche Beschreibung der Figuren

Die vorliegende Erfindung wird im Folgenden anhand der Zeichnungen der Figuren detailliert beschrieben werden. Dabei werden gleiche Bezugszeichen für ähnliche oder gleiche Bestandteile verwendet und auf eine wiederholte Beschreibung in den unterschiedlichen Ausführungsbeispielen kann verzichtet werden, um Redundanzen zu vermeiden.

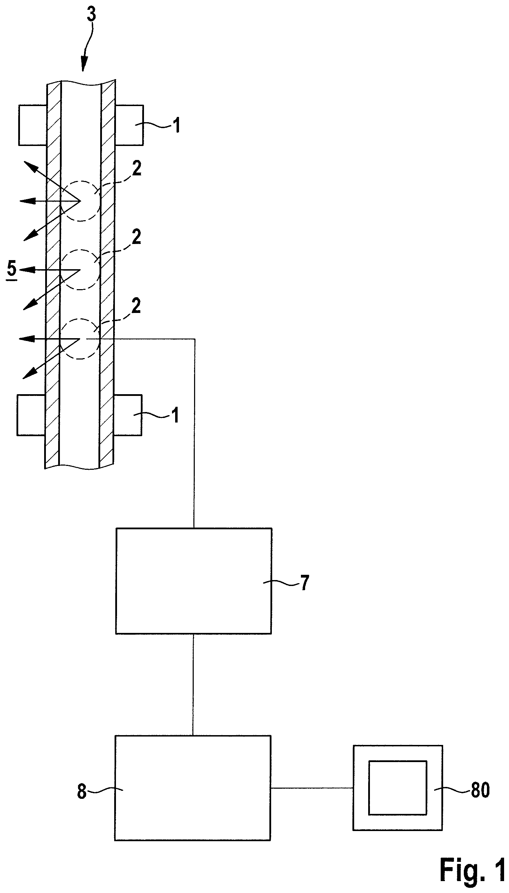

Figur 1 zeigt schematisch einen Systemschlauch 3 eines medizinischen Geräts, welcher als Einmalartikel ausgebildet ist. Dieser Systemschlauch 3 ist in Halterungen 1 an dem Hauptteil des medizinischen Geräts eingeklemmt und wird so gehalten.

Eine Signalisiervorrichtung 2 ist vorgesehen, welche in dem medizinischen Gerät so angeordnet ist, dass die zu bedienende Komponente, nämlich in diesem Fall der Systemschlauch 3, ein Signal aussendet. Dieses Signal ist hier in Form des Lichtes, welches schematisch durch die Pfeile 5 angedeutet ist und welches von dem Systemschlauch 3 ausgeht, dargestellt. Entsprechend signalisiert die Signalisiervorrichtung 2 dem Benutzer, dass ein Bedieneingriff am Systemschlauch 3 notwendig ist. Ein solcher Bedieneingriff kann beispielsweise das notwendige Austauschen des Systemschlauches 3 sein.

Die Signalisiervorrichtung 2 ist in dem gezeigten Ausführungsbeispiel in Form von Lichtquellen, bevorzugt LEDs, ausgebildet, welche in dem medizinischen Gerät so angeordnet sind, dass sie derart hinter dem Systemschlauch 3 angeordnet sind, dass bei Aktivierung der Lichtquellen 2 der Systemschlauch 3 so beleuchtet wird, dass er durch die diffuse Streuung des emittierten Lichtes an der Oberfläche des Schlauches sowie durch die Transmission des Lichtes durch den Schlauch zumindest teilweise selbst zu leuchten scheint und der Schlauch 3 entsprechend ein Signal an den Benutzer ausgibt.

Die Signalisiervorrichtung 2 in Form der Lichtquellen ist mit einer Steuervorrichtung 7 verbunden. Die Steuervorrichtung 7 steuert die Signalisiervorrichtung 2 derart an, dass die Signalisiervorrichtung immer dann aktiviert wird, wenn ein Bediener darauf hingewiesen werden soll, dass mit der durch die Signalisiervorrichtung 2 zu signalisierenden Komponente, in diesem Fall dem Systemschlauch 3, eine Aktion durchgeführt werden soll. Beispielsweise kann in dem in Figur 1 gezeigten Fall signalisiert werden, dass der Systemschlauch 3 nicht korrekt in die Halterungen 1 eingelegt wurde, oder es kann signalisiert werden, dass der Systemschlauch 3 aus den Halterungen 1 entfernt werden soll, oder es kann signalisiert werden, dass der Systemschlauch 3 ausgetauscht werden soll und entsprechend aus den Halterungen 1 entnommen werden soll.

Bevorzugt ist die Signalisiervorrichtung 2 beziehungsweise die Steuervorrichtung 7 mit einem Systemmonitor 8 des medizinischen Geräts in Kommunikation verbunden. Der Systemmonitor 8 des medizinischen Geräts überwacht dieses sowie die Zustände in dem medizinischen Gerät und kann entsprechend bei Vorliegen eines Alarmzustandes beziehungsweise bei Vorliegen einer Situation, in welcher ein Bedienereingriff notwendig ist, die Steuervorrichtung entsprechend darüber informieren.

Der Systemmonitor 8 kann weiterhin an einer weiteren Benutzerschnittstelle, beispielsweise einem Bildschirm 80, Informationen an den Benutzer ausgeben, beispielsweise bezüglich des derzeitigen Status des medizinischen Geräts und/oder bezüglich weiterer notwendiger Benutzerschritte. Durch die gleichzeitige Ausgabe von Informationen, beispielsweise Textinformationen oder Piktogrammen oder einer Kombination derer auf dem Bildschirm 80 durch den Systemmonitor 8, und die gleichzeitige Signalisierung einer zu bedienenden Komponente über die Signalisiervorrichtung 2 über die Steuervorrichtung 7 hinweg, wird dem Benutzer zum einen eine Erklärung der durchzuführenden Aktion geliefert, und zum anderen wir der Benutzer direkt intuitiv direkt zu der Komponente geführt, an welcher ein Eingriff vorgenommen werden muss.

Die Art der Signalisiervorrichtungen 2 kann dabei angepasst sein an die jeweiligen Bedürfnisse und baulichen Gegebenheiten des medizinischen Geräts.

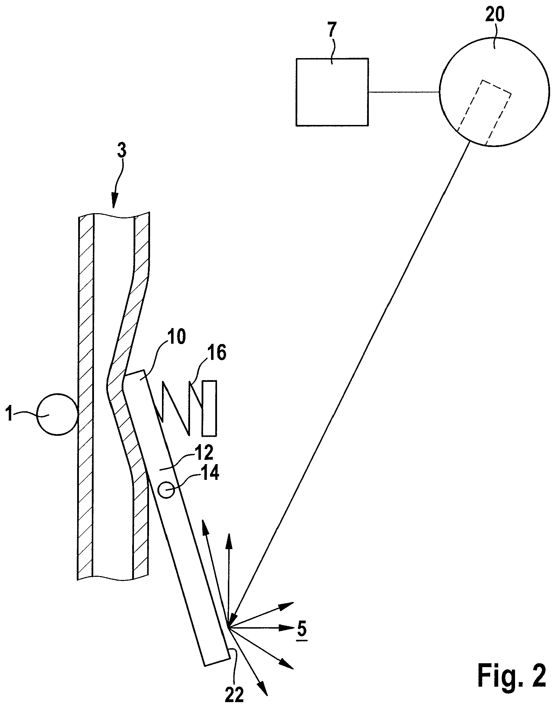

Beispielsweise wird in Figur 2 wiederum ein Systemschlauch 3 zwischen einem Lagerpunkt/Haltepunkt 1 und einer Klemme 10, also einer manuell betätigten Klemmvorrichtung eingeklemmt. Ein Klemmhebel 12, der um eine Drehachse 14 herum verschwenkbar ist und mittels einer Feder 16 in Klemmrichtung vorgespannt ist, klemmt entsprechend den Systemschlauch 3 ein.

Sollte an dieser Klemmvorrichtung beziehungsweise diesem Klemmhebel ein Bedienereingriff notwendig sein, so wird dies über eine Signalisiervorrichtung signalisiert. Die Signalisiervorrichtung 2 ist hier mehrteilig ausgeführt und umfasst einen Projektor 20 und eine Projektionsoberfläche 22, die an dem Klemmhebel 12 vorgesehen ist. Entsprechend wird der Projektor 20 über die Steuervorrichtung 7 angesteuert, wenn an den Benutzer signalisiert werden soll, dass die Komponente in Form der Klemmvorrichtung 12 betätigt werden soll. Der Projektor 20 projiziert dann ein entsprechendes Symbol oder lediglich eine Beleuchtung auf der Projektionsoberfläche 22 des Klemmhebels 12, so dass wiederum Lichtsignale 5 emittiert werden, die vom Benutzer wahrgenommen werden können. Der Benutzer wird entsprechend unmittelbar zur Betätigung des Klemmhebels 12 geführt.

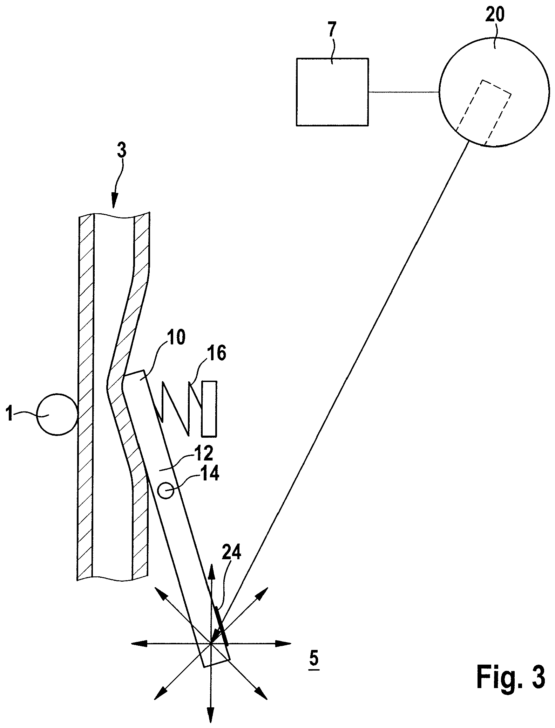

Figur 3 zeigt eine Variante der in Figur 2 gezeigten Vorrichtung. Der Klemmhebel 12 ist diesmal aus einem semitransparenten Material ausgebildet, derart, dass der Projektor 20, wenn er Licht auf die Komponente projiziert, diese quasi "von innen" zum Leuchten bringt, so dass wiederum Licht 5 von dem Klemmhebel 12 emittiert wird, welches beim Benutzer als Signal zur Signalisierung der zu bedienenden Komponente wahrgenommen wird. Bei der semitransparenten Komponente 12 kann es sich in diesem Beispiel auch um eine Komponente handeln, welche ein fluoreszierendes Material umfasst und der Projektor 20 kann entsprechend ein Lichtsignal in einem Wellenlängenbereich senden, welches das fluoreszente Material zur Fluoreszenz anregt, aber bevorzugt vom menschlichen Auge nicht erkannt wird. Entsprechend nimmt der Benutzer den vom Projektor 20 in den Klemmhebel 12 eingekoppelten Lichtstrahl nicht wahr, sondern lediglich das Fluoreszenzlicht, welches in Antwort darauf von dem Klemmhebel 12 emittiert wird.

Um das Einkoppeln des Lichtstrahls, der von dem Projektor 20 emittiert ist, in die zu bedienende Komponente, also den Klemmhebel 12, zu erleichtern, kann diese zumindest im Bereich der Lichteintrittsfläche mit einer Antireflexbeschichtung 24 versehen sein.

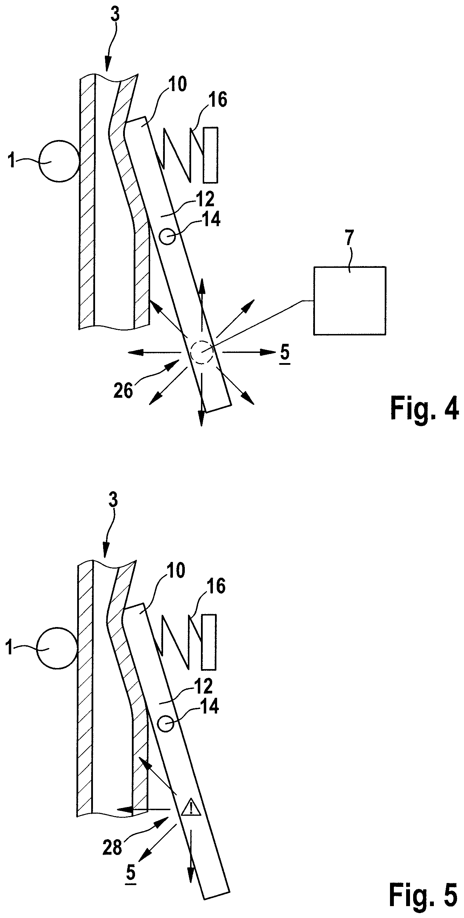

In dem in Figur 4 gezeigten Ausführungsbeispiel, welches vom mechanischen Aufbau her den in Figur 2 und 3 gezeigten Klemmhebeln entspricht, ist im Klemmhebel 12 selbst eine Lichtquelle 26 vorgesehen, beispielsweise in Form einer LED, welche über die Steuervorrichtung 7 wiederum genau dann angesteuert wird, wenn dem Benutzer signalisiert werden soll, dass die Klemmvorrichtung betätigt werden muss.

Figur 5 zeigt eine weitere Ausführungsform, bei welcher ein optisches Symbol 28 auf dem Klemmhebel 12 aufgebracht ist. Das Symbol 28 kann entweder aus einem fluoreszierenden Material aufgebracht sein, so dass es beginnt zu leuchten, wenn es mit einem entsprechenden Licht bestrahlt wird. Das optische Symbol kann in Form einer Brechungsindexänderung aufgebracht sein, beispielsweise durch das selektive Aufbringen einer Antireflexbeschichtung, oder es kann als semitransparentes oder opakes Material aufgebracht sein, derart, dass bei einer Beaufschlagung des Klemmhebels 12 mit Licht das Symbol 28 für den Benutzer unterscheidbar in Erscheinung tritt. Im Falle der Fluoreszenz wird das Material entsprechend fluoreszieren, im Falle eines semitransparenten Materials wird die Markierung heller leuchten als der Rest des Klemmhebels, im Falle eines opaken Materials wird der Klemmhebel 12 heller leuchten als das Symbol 28. Wichtig in diesem Zusammenhang ist lediglich, dass der Benutzer ein optisches Symbol 28 auf dem Klemmhebel 12 klar unterscheiden kann.

In einer bevorzugten Variante der in Figur 5 gezeigten Ausführungsform können auch unterschiedliche optische Symbole 28 aufgebracht sein, bevorzugt sogar am gleichen Ort der Komponente. Diese unterschiedlichen optischen Symbole können beispielsweise durch fluoreszierende Materialien unterschiedlicher Eigenschaften derart aufgebracht sein, dass, wenn die Komponente 12 mit einem ersten Licht einer ersten Wellenlänge bestrahlt oder angeregt wird, ein erstes Symbol leuchtet und wenn die Komponente 12 mit einem zweiten Licht beziehungsweise einem Licht einer zweiten Wellenlänge bestrahlt, die zweite Komponente aufscheint.

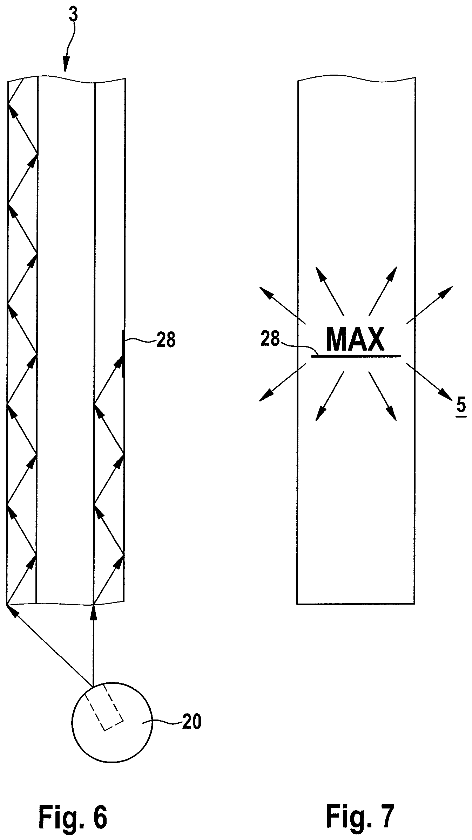

Die Figuren 6 und 7 zeigen schematisch einen Systemschlauch 3 in Form eines transparenten oder semitransparenten Einmalschlauches, in welchen mittels eines Projektors 20 Licht eingekoppelt wird. Es ist bekannt, dass das Licht, welches unter einem bestimmten Winkel in ein transparentes Material eingekoppelt wird, an den jeweiligen Grenzflächen total reflektiert wird und sich heraus der in Figur 6 auf der linken Seite gezeigte Lichtleitereffekt ergibt. Durch Bearbeitung der jeweiligen Oberfläche durch eine Brechungsindexänderung kann das in dem Lichtleiter geführte Licht wieder ausgekoppelt werden. Dies ist insbesondere in Figur 7 gezeigt. Diese Brechungsindexänderung zur Auskopplung eines optischen Symbols 28 kann beispielsweise durch das Aufbringen von einem Antireflexmaterial auf der Oberfläche des Einmalschlauches bewerkstelligt werden. Weiterhin kann durch einfaches Einfräsen beziehungsweise Einätzen die Oberfläche derart aufgeraut werden, dass der Auftreffwinkel keine Totalreflektion mehr ermöglicht und entsprechend ein Teil des Lichtes ausgekoppelt wird.

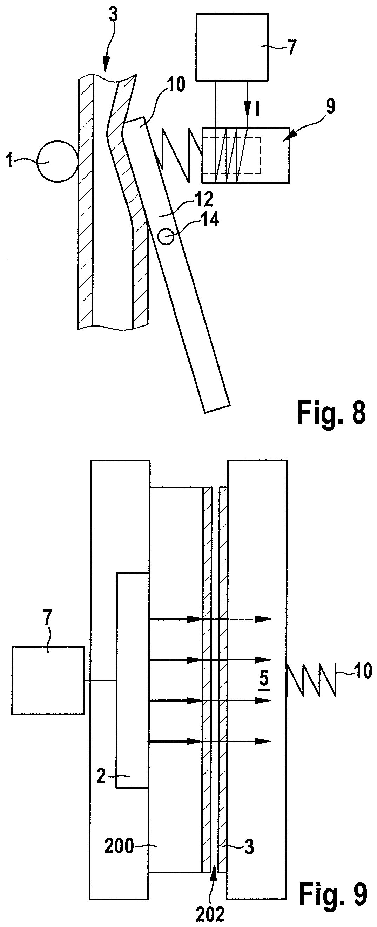

In Figur 8 ist schematisch eine weitere Variante der Klemmvorrichtung 12 gezeigt, bei welcher ein elektrodynamischer Wandler 9, der von einem Strom I durchflossen wird, als Signalisiervorrichtung verwendet wird. Entsprechend kann von der Steuervorrichtung 7 aus ein Strom I an den elektrodynamischen Wandler 9 dann ausgegeben werden, wenn eine Signalisierung stattfinden soll. Der Benutzer spürt ein Vibrieren und kann, je nach Frequenz des Stromes I, auch ein akustisches Signal wahrnehmen. Entsprechend kann in dieser Variante eine haptische und/oder akustische Signalisierung der zu bedienenden Komponente stattfinden.

Figur 9 zeigt eine weitere Ausführungsform, bei welcher eine Lichtquelle 2 durch eine transparente Abdeckung 200 hindurch und durch einen Luftspalt 202 in einen Einmalartikel 3 hereinstrahlt, der von einer Klemmvorrichtung 10 gehalten wird. Die Beleuchtungsvorrichtung 2 durchstrahlt entsprechend den Einmalartikel 3 und wird von der Steuervorrichtung 7 entsprechend angesteuert. Der Einmalartikel 3 kann bevorzugt eine die Transmission fördernde Beschichtung, beispielsweise eine Antireflexbeschichtung, aufweisen, so dass das Durchstrahlen mit einer hohen Effizienz stattfinden kann.

Zum Aussenden eines Signals von einem Einmalartikel aus ist bevorzugt an dem Einmalartikel eine Schnittstelle zum Einkoppeln von Licht vorgesehen, in welches Licht in den Einmalartikel eingeleitet werden kann und dann innerhalb des Einmalartikels, der in diesem Fall quasi als Lichtleiter fungiert, weitergeführt wird und an einer im Einmalartikel spezifizierten Stelle wieder sichtbar austritt, beispielsweise durch Behandlung der Oberfläche des Lichtleiters zur Veränderung des Brechungsindexes. Die entsprechenden Grenzflächen, beispielsweise an der Schnittstelle beziehungsweise der Austrittsstelle können mit einer Antireflexbeschichtung an dem Einmalartikel versehen sein. An dem Einmalartikel können halbtransparente Markierungen vorgesehen sein, welche durch das in den Einmalartikel eingekoppelte Licht zum Leuchten angeregt werden. Bevorzugt können solche halbtransparenten Markierungen derart ausgeführt sein, dass sie nur bei unterschiedlichen Wellenlängen zum Leuchten angeregt werden und entsprechen das Vorsehen mehrerer solcher Markierungen durch Einkoppeln von Licht unterschiedlicher Wellenlängen selektiv angesteuert werden können.

Die Signalisiervorrichtung kann auch eine nicht sichtbare Lichtquelle umfassen, welche bei der Komponente auf ein fluoreszierendes Medium trifft, um an der Komponente durch die Fluoreszenz sichtbares Licht zu emittieren.

## Patentansprüche

1. Blutbehandlungsvorrichtung mit einer Halterung (1) zum Halten eines als Einmalartikel ausgebildeten Systemschlauchs (3) und mit einer Vorrichtung zum Unterstützen eines Bedieners bei der Bedienung der Blutbehandlungsvorrichtung, wobei die Vorrichtung eine Signalisiervorrichtung (2) zum Signalisieren einer erforderlichen Bedienung des Systemschlauchs (3) umfasst, wobei die Vorrichtung eine Steuervorrichtung (7) zur Ansteuerung der Signalisiervorrichtung (2) in Abhängigkeit von der durchzuführenden Bedienung und unabhängig von einer Benutzeranforderung aufweist, und **gekennzeichnet dadurch** das die Signalisiervorrichtung (2) eine durch die Steuervorrichtung (7) ansteuerbare und hinter dem Systemschlauch (3) angeordnete Lichtquelle umfasst, welche den Systemschlauch (3) bei einer Aktivierung der Lichtquelle durch die Steuervorrichtung (7) zumindest teilweise durchleuchtet, um ein optisches Signal von dem Systemschlauch (3) aus auszusenden.

2. Blutbehandlungsvorrichtung gemäß Anspruch 1, wobei die Lichtquelle eine hinter dem Systemschlauch (3) angeordnete LED umfasst.

3. Blutbehandlungsvorrichtung gemäß Anspruch 1 oder 2, wobei die Steuervorrichtung (7) mit einem Systemmonitor (8) zur Überwachung der Blutbehandlungsvorrichtung verbunden ist und bevorzugt mit dem Systemmonitor korreliert ist.

4. Blutbehandlungsvorrichtung mit einer Klemmvorrichtung zum Einklemmen eines Systemschlauchs (3), wobei die Klemmvorrichtung einen semitransparenten Klemmhebel (12) aufweist und eine Vorrichtung zum Unterstützen eines Bedieners bei der Bedienung der Blutbehandlungsvorrichtung vorgesehen ist, wobei die Vorrichtung eine Signalisiervorrichtung (2) zum Signalisieren einer erforderlichen Bedienung des Klemmhebels (12) umfasst, wobei
die Vorrichtung eine Steuervorrichtung (7) zur Ansteuerung der Signalisiervorrichtung (2) in Abhängigkeit von der durchzuführenden Bedienung und unabhängig von einer Benutzeranforderung aufweist, und wobei
die Signalisiervorrichtung (2) eine durch die Steuervorrichtung (7) ansteuerbare und in dem Klemmhebel (12) angeordnete Lichtquelle (26) umfasst, welche bei einer Aktivierung der Lichtquelle (26) durch die Steuervorrichtung (7) den Klemmhebel (12) zum Leuchten bringt.

5. Medizinisches Gerät gemäß Anspruch 4, wobei die Lichtquelle eine in dem Klemmhebel (12) angeordnete LED umfasst.

6. Medizinisches Gerät gemäß Anspruch 4 oder 5, wobei die Steuervorrichtung (7) mit einem Systemmonitor (8) zur Überwachung der Blutbehandlungsvorrichtung verbunden ist und bevorzugt mit dem Systemmonitor korreliert ist.

## Claims

1. Blood treatment apparatus with a mounting (1) for holding a single-use system tube (3) and with a device for supporting an operator in operating the blood treatment apparatus, wherein the device comprises a signalling device (2) for signalling a necessary operation of the system tube (3), wherein the device comprises a control device (7) for controlling the signalling device (2) in dependency of the operation to be performed and independent of an operator request, **characterized in that**
the signalling device (2) comprises a light source which is controllable by the control device (7) and arranged behind the system tube (3), wherein the light source at least partially illuminates the system tube (3) when the light source is activated by the control device (7) to send out an optical signal from the system tube (3).

2. Blood treatment apparatus according to claim 1, wherein the light source comprises an LED arranged behind the system tube (3).

3. Blood treatment apparatus according to claim 1 or 2, wherein the control device (7) is connected to a system monitor (8) for monitoring the blood treatment apparatus and preferably is correlated with the system monitor.

4. Blood treatment apparatus with a clamping device for clamping a system tube (3), wherein the clamping device comprises a semi-transparent clamping lever (12) and a device for supporting an operator in operating the blood treatment apparatus is provided, wherein the device comprises a signalling device (2) for signalling a necessary operation of the clamping lever (12), wherein
the device comprises a control device (7) for controlling the signalling device (2) in dependency of the operation to be performed and independent of an operator request, and wherein
the signalling device (2) comprises a light source (26) which is controllable by the control device (7) and arranged in the clamping lever (12), wherein the light source illuminates the clamping lever (12) when the light source (26) is activated by the control device (7).

5. Medical device according to claim 4, wherein the light source comprises an LED arranged in the clamping lever (12).

6. Medical device according to claim 4 or 5, wherein the control device (7) is connected to a system monitor (8) for monitoring the blood treatment apparatus and preferably is correlated with the system monitor.

## Revendications

1. Dispositif de traitement du sang avec une fixation (1) pour tenir un tuyau de système (3) conçu sous forme d'article à usage unique et avec un dispositif pour assister un opérateur lors de l'utilisation du dispositif de traitement du sang, le dispositif comprenant un dispositif de signalisation (2) pour signaler une manipulation nécessaire du tuyau de système (3),
le dispositif comportant un dispositif de commande (7) pour commander le dispositif de signalisation (2) en fonction de la manipulation à effectuer et indépendamment d'une demande d'utilisateur,
**caractérisé en ce que** le dispositif de signalisation (2) comprend une source de lumière qui est commandable par le dispositif de commande (7) et agencée derrière le tuyau de système (3) et qui éclaire au moins partiellement à travers le tuyau de système (3) lors d'une activation de la source de lumière par le dispositif de commande (7) pour émettre un signal optique à partir du tuyau de système (3).

2. Dispositif de traitement du sang selon la revendication 1, dans lequel la source de lumière comprend une LED agencée derrière le tuyau de système (3).

3. Dispositif de traitement du sang selon la revendication 1 ou 2, dans lequel le dispositif de commande (7) est relié à un moniteur de système (8) pour surveiller le dispositif de traitement du sang et est corrélé de préférence avec le moniteur de système.

4. Dispositif de traitement du sang avec un dispositif de serrage pour serrer un tuyau de système (3), le dispositif de serrage comportant un levier de serrage semi-transparent (12) et un dispositif étant prévu pour assister un opérateur lors de l'utilisation du dispositif de traitement du sang, le dispositif comprenant un dispositif de signalisation (2) pour signaler une manipulation nécessaire du levier de serrage (12),
le dispositif comportant un dispositif de commande (7) pour commander le dispositif de signalisation (2) en fonction de la manipulation à effectuer et
indépendamment d'une demande d'utilisateur et
le dispositif de signalisation (2) comprenant une source de lumière (26) qui est commandable par le dispositif de commande (7) et agencée dans le levier de serrage (12) et qui amène le levier de serrage (12) à être lumineux lors d'une activation de la source de lumière (26) par le dispositif de commande (7).

5. Appareil médical selon la revendication 4, dans lequel la source de lumière est une LED agencée dans le levier de serrage (12).

6. Appareil médical selon la revendication 4 ou 5, dans lequel le dispositif de commande (7) est relié à un moniteur de système (8) pour surveiller le dispositif de traitement du sang et est corrélé de préférence avec le moniteur de système.
